Europäisches Patentamt

European Patent Office

Office européen des brevets

(19)

(11) Publication number: **0 357 330**
**A2**

(12) # EUROPEAN PATENT APPLICATION

(21) Application number: **89308548.0**

(22) Date of filing: **23.08.89**

(51) Int. Cl.⁵: **A 61 M 25/08**

(30) Priority: **01.09.88 US 239507**

(43) Date of publication of application:
**07.03.90 Bulletin 90/10**

(84) Designated Contracting States: **DE FR GB**

(71) Applicant: **BAXTER INTERNATIONAL INC.**
**One Baxter Parkway**
**Deerfield, IL 60015 (US)**

(72) Inventor: **Cohen, Donald M.**
**394 Knoll Glen Street**
**Irvine, CA 92715 (US)**

**Judge, Orvil L.**
**840 Shaffer Street**
**Orange, CA 92667 (US)**

(74) Representative: **MacGregor, Gordon et al**
**ERIC POTTER & CLARKSON 14 Oxford Street**
**Nottingham, NG1 5BP (GB)**

The title of the invention has been amended (Guidelines for Examination in the EPO, A-III, 7.3).

(54) **Catheter feed mechanism.**

(57) A feed mechanism includes a housing dimensioned and arranged to be grasped in a hand of a user. The housing defines a passage in which to place a portion of a catheter having inner and outer members, such as an everting balloon catheter having inner and outer catheters such that the user may advance the inner catheter within the outer catheter for balloon eversion purposes. Actuator components on the housing that are operable by movement of the hand of the user while the housing is being grasped in the hand enables the user to advance the inner catheter within the outer catheter using just the one hand. One embodiment includes an actuator to be actuated by the thumb of the hand of the user while the housing is being grasped in the hand and components on the housing responsive to the actuator being actuated for advancing the inner catheter within the outer catheter. The housing may include first and second sections that cooperatively define the passage, which first and second sections can be removably attached together over the portion of the catheter for purposes of placing the portion of the catheter within the passage. The passage may be dimensioned and arranged so that the housing engages a proximal connector on a proximal end portion of the outer catheter.

FIG.2

EP 0 357 330 A2

## Description

## Balloon Catheter Feed Mechanism

### Background of the Invention

### Technical Field

This invention relates generally to balloon catheters, and more particularly to a mechanism for advancing an inner catheter within an outer catheter for balloon eversion purposes.

### Background Information

Some balloon catheters include a balloon that is fed into position by advancing an inner catheter within an outer catheter. This causes the balloon to evert out of the distal end of the outer catheter and through a stenosis in the interior lumen of an artery or through a strictured area otherwise located in the human body. Once in position and inflated, the balloon reforms, enlarges, or dilatates the strictured area.

An existing balloon catheter for gastrointestinal dilatation or G.I. dilatation catheter, for example, such as the everting, thru-lumen, balloon catheter sold under the trademark TRANSLINEAR by American Edwards Laboratories, allows access into and dilatation of a strictured or tortuous area of the gastrointestinal system, such as a narrowed areas of the esophagus, pylorus, duodenum, or biliary tree. The device includes an inner catheter within an outer catheter that defines a balloon inflation lumen. It also includes an elastomeric, minimally distensible, polyethylene balloon connected to the distal ends of the inner and outer catheters in an inverted, deflated position.

Advancing the tip of the G.I. dilatation catheter to a stricture area positions the balloon adjacent the stricture. Injecting fluid through the balloon inflation lumen into the balloon and advancing the inner catheter within the outer catheter causes the balloon to evert from the distal end of the outer catheter and past the stricture as the balloon inflates to a predetermined length and diameter, thereby achieving access and dilatation.

The balloon is connected to the inner and outer catheters in such a manner as to create a thru-lumen after balloon eversion. Thus, the device may be used for guidewire placement and infusion or drainage of fluids before or after balloon eversion. A radiopaque marker at the catheter tip enhances fluoroscopic visualization, while a Y-adapter or proximal connector at the proximal end of the catheter has two extensions that allow for balloon inflation through one extension and guidewire passage and infusion or drainage of fluids through the other.

First, the patient is cleaned, draped, and anesthetized in a manner consistent with standard gastrointestinal access and dilation procedures and the catheter insertion site is prepared in the standard manner. Next, the catheter is advanced to the strictured area under endoscopic and/or fluoroscopic visualization.

When the catheter tip is just proximal to the area requiring access and/or dilation, a pressurized fluid is injected into the balloon inflation lumen and the inner catheter is manually advanced within the outer catheter to evert the balloon through the stricture. This is done until the balloon is completely everted and inflated, the duration of balloon inflation and the inflation pressure being determined by the physician for each case within prescribed limits. This dilatates the stricture. Use is followed by aspirating fluid from the balloon and withdrawing the catheter.

Thus, use of a balloon catheter such as the everting, thru-lumen balloon catheter described above is very convenient and effective in many respects. Among other things, the balloon rolls out of the distal end of the outer catheter and against the stricture instead of scraping against it. This results in less stress to body tissue because there is less tendency to rub off the mucosal lining of the gastrointestinal system or the intimal lining of a blood vessel. Additionally, advancement of an everting balloon permits access through ducts sufficiently tortuous or constricted so as to completely inhibit cannulation by most any other means know in the art, including the use of super selective or steerable guidewires or catheters.

However, certain problems need to be overcome. Consider, for example, the procedure of advancing the inner catheter within the outer catheter in order to evert the balloon out of the distal end of the outer catheter. This is often accomplished by first inserting a stiff wire or stylet into the proximal end portion of the inner catheter to stiffen the inner catheter body in order to facilitate its advancement within the outer catheter. Stiffening is helpful because the inner catheter body may otherwise buckle under the force necessary to advance it against the inverted balloon, frictional forces between the inner and outer catheters, and frictional forces between the inner catheter and an adjustable O-ring arrangement on the proximal connector that allows advancement of the inner catheter without fluid leakage from the balloon inflation lumen.

But, the physician has several things to do, such as holding an endoscope in front of his eyes while grasping the proximal connector of the balloon catheter, advance the inner catheter, and maintain pressure within the balloon lumen. Alternatively, the physician might delegate one or more of these tasks to another member of the operating team.

In any case, inner catheter advancement within the outer catheter for purposes of balloon eversion is somewhat awkward and may therefore result in ineffective treatment for the patient. Consequently, it is desirable to have some way of simplifying this procedure.

### Summary of the Invention

This invention solves the problems outlined above with a hand-held, thumb-operated feed mechanism that mounts on the proximal connector. The physician grasps the feed mechanism between the fingers and palm of one hand while using the thumb of that hand to pump an actuator on the mechanism that advances the inner catheter in a series of small steps, one step for each push of the actuator. Thus, only one hand is required.

Generally, a feed mechanism constructed according to a major aspect of the invention includes a housing dimensioned and arranged to be grasped in a hand of a user. The housing defines a passage in which to place a portion of a balloon catheter having inner and outer catheters such that the user must advance the inner catheter within the outer catheter for balloon eversion purposes.

Actuator components are included on the housing. They are operable by movement of the hand of the user while the housing is being grasped in the hand. This enables the user to advance the inner catheter within the outer catheter using just one hand.

According to another aspect of the invention, the housing includes first and second sections that cooperatively define the passage. They can be removably attached over the balloon catheter in order to place the balloon catheter in the passage.

According to yet another aspect of the invention, the actuator components include an actuator button arranged to be pumped with the thumb. This causes the inner catheter to advance in a series of small steps.

According to still another aspect of the invention, there is provided a balloon catheter assembly that includes a balloon catheter and a feed mechanism as described above. The balloon catheter may be a gastrointestinal dilatation catheter, for example.

In line with the above, a method of advancing an inner catheter within an outer catheter for balloon eversion purposes includes providing a feed mechanism as described above, placing the portion of the balloon catheter in the passage, grasping the housing in a hand, and actuating the actuator means with the hand to evert the balloon in a series of steps.

The above mentioned and other objects and features of this invention and the manner of attaining them will become apparent, and the invention itself will be best understood, by reference to the following description taken in conjunction with the accompanying illustrative drawings.

Brief Description of the Drawings

FIGURE 1 of the drawings is a perspective view of a feed mechanism constructed according to the invention;

FIGURE 2 is an exploded view of the feed mechanism showing the first and second sections of the housing in a position to be mounted on the proximal connector of an everting balloon catheter;

FIGURE 3 is a top view of the mechanism;

FIGURE 4 is a bottom view of the mechanism;

FIGURE 5 is an enlarged view of the first section of the feed mechanism taken on line 5-5 of Fig. 1 with the catheter in position; and

FIGURE 6 is an enlarged cross sectional view of the feed mechanism taken on line 6-6 of Fig. 1, with the catheter added to further illustrate positioning.

Description of the Preferred Embodiments

Referring now to the drawings, there is shown a feed mechanism 10 constructed according to the invention. Generally, the mechanism 10 includes a housing 11 dimensioned and arranged to be grasped in a hand of a user. It may take any of various shapes and sizes for this purpose and be constructed of a suitable material, such as an injection molded thermoplastic material.

The housing 11 includes first and second sections 12 and 13 that cooperatively define a passage 14 (Figs. 3 and 4) in which to place a portion of a catheter, such as the gastrointestinal dilatation, everting balloon catheter 15 illustrated in Fig. 2. This a known type of everting balloon catheter having inner and outer catheters 16 and 17 that a user just manipulate for balloon eversion purposes. Of course, the mechanism 10 can be used with other catheters as well where it is desired to advance an inner member within an outer member.

The outer catheter 17 extends from a proximal end portion 18 to a distal end portion 19 in which is disposed a balloon in an inverted position as is known in the art (not shown), the outer catheter 17 defining a suitable balloon inflation lumen (not shown) through which fluid is communicated to the balloon for inflation purposes. A Y-adapter or proximal connector 20 attached to the proximal end portion 18 provides a first extension 21 through which to introduce a fluid into the balloon inflation lumen and a second extension 22 through which to feed the inner catheter 16 into the outer catheter 17. The proximal connector 20 may utilize suitable known structure, such as a Touhy-Borst valve for this purpose.

In order to facilitate advancement of the inner catheter 16, a stylet 23 is inserted in a proximal end portion 24 of the inner catheter 16 (Fig. 2). The stylet 23 includes a cap 25 to which is attached a stiff wire 26 that fits within the inner catheter 16. A portion of the inner catheter 16 is shown removed in Fig. 2 to show the wire 16 extending within it. Alternatively, a guidewire may be substituted for the stylet.

According to existing procedures, the user grasps the proximal connector with the fingers of one hand and the stylet-stiffened inner catheter 16 with the fingers of the other hand. Then, while applying pressure to the balloon inflation lumen, the user advances the inner catheter 16 further into the outer catheter 17 for balloon eversion purposes. So that the applied pressure is exactingly applied and so that the operator need not employ marginally effective means when superior means are available, an inflation device may be used such as the pressure source sold by American Edwards Laboratories under the trademark INFLATION PRO.

With the mechanism 10, however, only one hand is needed to advance the inner catheter 16. First, the user separates the first and second sections 12 and 13. Next, the user reassembles the first and second sections 12 and 13 over the everting balloon catheter 15 utilizing a bottom plate 27 and a top plate 28 that are already attached to the second section 13 by suitable means such as screws 29. The bottom plate 27 and the top plate 28 provide a cantilever spring latch mechanism to retain the first section 12 fixed in contact with the second section 13.

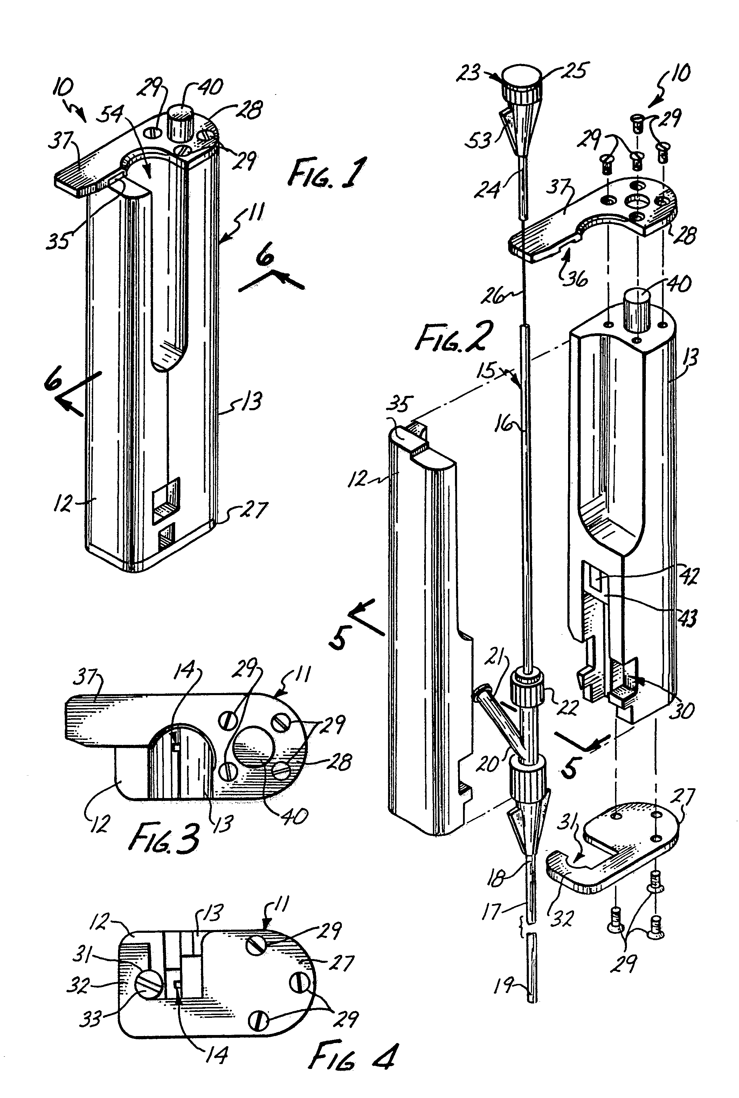

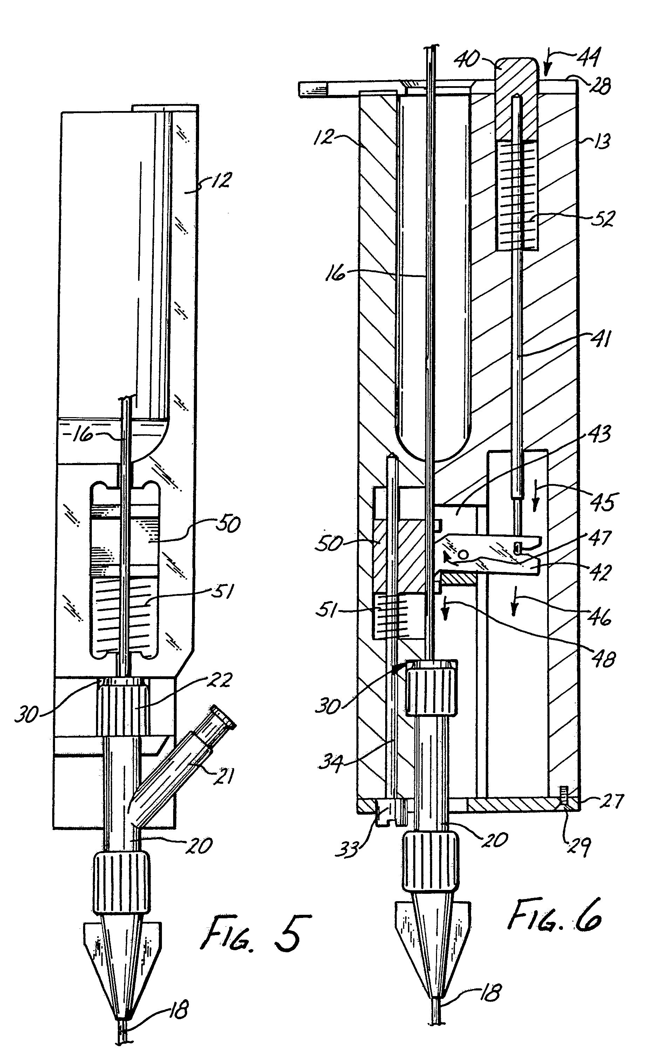

The first and second sections 12 and 13 are joined or separated by elastically bending the leg 37 of the top plate 28 in an upward direction to allow the first and second sections 12 and 13 to mate. Once the first and second section 12 and 13 are brought together to the position illustrated in Fig. 1 and the leg 37 is released, the first and second sections 12 and 13 are fastened firmly together. This is done so that the proximal connector 20 lies within a recess 30 and the proximal end portion 24 of the inner catheter 16 extends beyond the top plate 28 in the position illustrated in Fig. 2.

This results in the housing 11 engaging the proximal connector 20, and thereby the proximal end portion 19 of the outer catheter 17, in order to inhibit or prevent movement of the proximal connector 20 relative to the housing 11.

More specifically, the bottom plate 27 is placed so that an indentation or recess 31 in a leg 32 of the bottom plate 27 engages the head 33 of a screw 34 (Figs. 3, 4 and 6). Next, the first and second sections 12 and 13 are brought fully together over the everting balloon catheter 15. As this is done, the top plate 28 is snapped into position over a ridge 35 on the housing 11 so that the ridge 35 is received in an indentation or recess 36 in a leg 37 of the top plate 28 (Figs. 1-3). This locks the first and second sections 12 and 13 together, with the leg 37 overhanging the first section 12 so that the user can push against it when desired in order to unlock and separate the two sections 12 and 13 for purposes of removing the everting balloon catheter 15.

The mechanism 10 includes actuator means on the housing 11 that is operable by movement of the hand of the user while the housing is being grasped in the hand for enabling the user to advance the inner catheter 16 within the outer catheter 17 using just one hand. A finger-operated actuator in the form of a thumb-operated pushbutton 40 is provided in the illustrated embodiment for this purpose (Figs. 1-3 and 6). It is arranged so that it can be actuated while the housing 11 is being grasped in the hand, typically with the fingers of the users hand bearing against the first section 12, the palm of the hand bearing against the second section 13, and the thumb elevated in a position to depress the pushbutton 40. Of course, other mechanical arrangements can be utilized within the broader inventive concepts disclosed, including actuation by a finger other than the thumb or by squeezing the housing, for example.

The mechanism 10 includes means on the housing 11 responsive to the pushbutton 40 being actuated for advancing the inner catheter 16 within the outer catheter 17. This is accomplished with a linkage 41 that couples the pushbutton 40 to a dog 42. The dog 42 is pivotally mounted within the housing 11 on a carriage 43 so that when the pushbutton 40 is actuated (i.e., pushed in the direction of an arrow 44 in Fig. 6), the linkage 41 travels in the direction of an arrow 45 to cause the dog 42 to travel in the direction of an arrow 46 and pivot in the direction of an arrow 47. This action causes the dog 42 to bear against the inner catheter 16 and travel with the carriage 43 in the direction of an arrow 48.

When the everting balloon catheter 15 is placed within the passage 14, the inner catheter 16 sets against a block 50 that is movably mounted within the housing 11 so that it can travel against a spring 51 (Figs. 5 and 6). When dog 42 bears against the inner catheter 16 in response to operation of the pushbutton 40, the inner catheter 16 is forced against the block 50 so that the inner catheter 16 is frictionally engaged between the dog 42 and the block 50.

As a result, the inner catheter 16 and the block 50 travel with the dog 42 and the carriage 43 in the direction of the arrow 48 when the pushbutton 40 is actuated. The amount of travel may be on the order of one-half inch, for example, with the spring 51 compressing and the inner catheter 16 advancing that distance (a single one-half inch step). When the pushbutton 40 is released, the spring 51 forces the block 50 to travel back in a direction opposite to the arrow 48 while a spring 52 (Fig. 6) forces the dog 42, the linkage 41, and the pushbutton 40 back, the dog 42 also pivoting back in a direction opposite to the arrow 47 so that the inner catheter 16 is released during the back travel.

Then, the pushbutton 40 can be actuated again to advance the inner catheter 16 another step. Thus, alternately pushing and releasing or pumping the pushbutton 40 causes the inner catheter 16 to advance within the outer catheter 17 in a series of small steps.

To use the mechanism 10 with the catheter 15, the everting balloon catheter 15 is first placed within the mechanism 10 as described above. Then, the patient is cleaned, draped, and anesthetized in a manner consistent with standard gastrointestinal access and dilation procedures and the catheter insertion site is prepared in the standard manner. Next, the everting balloon catheter 15 is advanced to the strictured area under endoscopic and/or fluoroscopic visualization according to existing procedures.

When the distal end portion 19 of the outer catheter 17 of the everting balloon catheter 15 is just proximal to the area requiring access and/or dilation, a pressurized fluid is injected into the balloon inflation lumen and the inner catheter 16 is manually advanced within the outer catheter 17 to evert the balloon through the stricture. This is done until the balloon is completely everted and inflated by the physician grasping the housing 11 in a hand and actuating the pushbutton 40 with the thumb of the hand to evert the balloon in a series of steps.

The physician may choose at any point during this procedure to cease advancement of the inner catheter 16 to either advance a guidewire through the inner catheter 16 or to infuse or withdraw fluid through the inner catheter 16. Should this occur while the inner catheter luer connector 53 lies within the body of the mechanism 10, the luer connector 53 would still be accessible by virtue of a cavity 54. This cavity is formed when the first and second sections 12 and 13 are joined and is provided for this purpose.

Thus, this invention overcomes problems of existing procedures with a hand-held, thumb-operated feed mechanism that mounts on the proximal connector so that only one hand is required.

Although an exemplary embodiment of the invention has been shown and described, many changes, modifications, and substitutions may be made by one having ordinary skill in the art without necessarily departing from the spirit and scope of the invention.

## Claims

1. A feed mechanism, comprising:
a housing dimensioned and arranged to be grasped in a hand of a user;
the housing defining a passage in which to place a portion of a catheter having inner and outer members such that the user must advance the inner member within the outer member; and
actuator means on the housing that is operable by movement of the hand of the user while the housing is being grasped in the hand for enabling the user to advance the inner member within the outer member using just one hand.

2. A mechanism as recited in Claim 1, wherein:
the catheter is an everting balloon catheter having inner and outer catheters such that the user must advance the inner catheter within the outer catheter for balloon eversion purposes.

3. A mechanism as recited in Claim 2, wherein the actuator means includes:
an actuator mounted on the housing in a position to be actuated by a finger of the hand of the user while the housing is being grasped in the hand; and
means on the housing responsive to the actuator being actuated for advancing the inner catheter within the outer catheter.

4. A mechanism as recited in Claim 3, wherein: the actuator is mounted on the housing in a position to be actuated by the thumb of the hand of the user.

5. A mechanism as recited in Claim 4, wherein: the actuator is actuated by pumping it with the thumb.

6. A mechanism as recited in Claim 3, wherein the means for advancing the inner catheter includes:
means for engaging the inner catheter frictionally in order to advance it a step each time the actuator is actuated.

7. A mechanism as recited in Claim 2, wherein the housing includes:
means for engaging the proximal end portion of the outer catheter to inhibit movement relative to the housing.

8. A mechanism as recited in Claim 7, wherein the everting balloon catheter includes an outer catheter extending between proximal and distal end portions of the outer catheter, a proximal connector mounted on the proximal end portion of the outer catheter, and an inner catheter that extends through the proximal connector into the outer catheter, and the means for engaging includes: a portion of the passage that is dimensioned and arranged so that the housing engages the proximal connector.

9. A mechanism as recited in Claim 1, wherein the housing includes:
first and second sections that cooperatively define the passage;
which first and second sections can be removably attached together over the portion of the catheter for purposes of placing the portion of the catheter within the passage.

10. A feed mechanism, comprising:
a housing dimensioned and arranged to be grasped in a hand of a user;
the housing defining a passage extending through the housing;
the passage being dimensioned and arranged to receive a portion of an everting balloon catheter having an outer catheter extending between proximal and distal end portions of the outer catheter, a proximal connector mounted on the proximal end portion of the outer catheter, and an inner catheter that extends through the proximal connector into the outer catheter;
means for engaging the proximal end portion of the outer catheter to inhibit movement relative to the housing;
an actuator mounted on the housing in a position to be actuated by a finger of the hand while the housing is being grasped in the hand; and
means on the housing responsive to the actuator being actuated for advancing the inner catheter within the outer catheter for balloon eversion purposes.

11. A everting balloon catheter assembly, comprising:
an everting balloon catheter having an outer catheter extending between proximal and distal end portions of the outer catheter, a proximal connector mounted on the proximal end portion of the outer catheter, and an inner catheter that extends through the proximal connector into the outer catheter;
a housing dimensioned and arranged to be grasped in a hand of a user, the housing defining a passage in which to place a portion of the everting balloon catheter; and
actuator means on the housing that is operable by movement of the hand of the user while the housing is being grasped in the hand for enabling the user to advance the inner catheter within the outer catheter using just one hand for balloon eversion purposes.

12. An assembly as recited in Claim 11, wherein:
the everting balloon catheter is a gastrointestinal dilatation catheter.

13. A method of advancing an inner catheter within an outer catheter of an everting balloon catheter for balloon eversion purposes, comprising:

providing a housing dimensioned and arranged to be grasped in a hand of a user, the housing defining a passage in which to place a portion of the everting balloon catheter and the housing including actuator means that is operable by movement of the hand of the user while the housing is being grasped in the hand for enabling the user to advance the inner catheter within the outer catheter using just one hand;

placing the portion of the everting balloon catheter in the passage;

grasping the housing in a hand; and

actuating the actuator means with the hand to evert the balloon in a series of steps.

14. A method as recited in Claim 13, wherein: the step of providing includes providing a housing having actuator means that is arranged to be actuated by movement of the thumb of the hand of the user.

15. A method as recited in Claim 13, wherein: the everting balloon catheter includes a proximal connector on a proximal end portion of the outer catheter; and

the passage is dimensioned and arranged so that the housing engages the proximal connector.

FIG. 1

FIG. 2

FIG. 3

FIG 4

FIG. 5

FIG. 6